# EUROPEAN PATENT APPLICATION

(11) **EP 2 526 924 A1**
(43) Date of publication of application: **28.11.2012**
(21) Application number: 11167112.9
(22) Date of filing: 23.05.2011
(51) Int. Cl.: A61K 9/00, A61K 31/13, A61M 15/00, B65D 83/52, A61K 31/135

(54) **A dose adjustable oral pump spray or aerosol spray containing rasagiline**

(71) Applicant: Deva Holding Anonim Sirketi, 34303 Istanbul (TR)
(72) Inventor: Haas, Philipp Daniel, 34303, ISTANBUL (TR); Sivasligil, Ramazan, 34303, ISTANBUL (TR); Koc, Fikret, 34303, ISTANBUL (TR); Kandemir, Levent, 34303, ISTANBUL (TR)

(57) **Abstract**

This invention directed to a pump spray or an aerosol spray formulation of rasagiline or pharmaceutically acceptable salt thereof for oral administration.

## Description

### Technical Field

This invention embraces a pump spray or aerosol spray formulation of rasagiline or pharmaceutically acceptable salt thereof for oral administration.

### Background Art

WO 2006/057912 A (TEVA PHARMACEUTICAL INDUSTRIES, LTD.) 01.06.2006 discloses orally disintegrating tablets of rasagiline.

### _{Summary of invention}

This invention provides oral spray of rasagiline or pharmaceutically acceptable salt thereof.

### Technical Problem

Orally disintegrating tablets are useful for elderly people who have difficulties in swallowing tablets. Such peoples are unwilling and/or unable to swallow tablets.

However in elderly people there is a group for which swallowing difficulties are persistant even tablet is orally disintegrating. Also one cannot be 100% sure that elderly people will swallow tablet which is disintegrated oral cavity since disintegration does not necessarily mean administration of tablet to gastrointestinal system.

Rasagiline or pharmaceutically acceptable salt thereof can be also used as adjunctive treatment with other disease treating and/or modifying agents for the treatment of Parkinson's disease. At the moment, rasagiline mesylate plus levodopa treatment is approved while other combinations are still in clinical phases. Rasagiline undergoes almost complete biotransformation in the liver prior to excretion. The metabolism of rasagiline proceeds through two main pathways: N-dealkylation and/or hydroxylation to yield 1aminoindan (AI), 3-hydroxy-N-propargyl-1 aminoindan (3-OH-PAI) and 3-hydroxy-1-aminoindan (3-OHAI). *In vitro* experiments indicate that both routes of rasagiline metabolism are dependent on the cytochrome P450 (CYP) system, with CYP1A2 being the major isoenzyme involved in rasagiline metabolism. Glucuronide conjugation of rasagiline and its metabolites, with subsequent urinary excretion, is the major elimination pathway. Since rasagiline is metabolized extensively with CYP1A2 isoenzyme, it should be carefully coadministered with other drugs (such as ciprofloxacin, theophylline) which also are metabolized by the same isoenzyme.

Due to its extensive metabolism in liver, when combination treatment, concomitant administration of other drugs or administration of rasagiline or pharmaceutically acceptable salt thereof to patients with mild hepatic impairment is required then dose titration of rasagiline or pharmaceutically acceptable salt thereof is a must.

### Solution to Problem

To solve the technical problems explained foregoing, it is invented that rasagiline is in the form of spray wherein dispenser emits an adjustable and predeterminable dose of liquid upon each actuation of the dispenser. Dispensing valve provides a quantity of pressurized liquid which can be adjustably selected. Thus patients are able to predetermine volume of the liquid.

### Description of embodiments

The object of this invention is to provide spray form of rasagiline or pharmaceutically acceptable salt thereof for oral administration.

Present invention employs a pump or aerosol spray dispenser to achieve dispensing of a predetermined quantity of liquid containing rasagiline or pharmaceutically acceptable salt thereof. Spray dispenser containing rasagiline provides a quantity of pressurized liquid wherein said quantity of liquid can be adjusted prior to dosing. Upon each actuation dispensing of constant amount of the liquid, as it is adjusted, is ensured.

In accordance with this invention, dosing indicators are placed on dispenser as 0.25 mg, 0.5 mg, 0.75 mg and 1 mg and each of doses are written. Each of indicators can be coloured to ensure patient compliance.

US 5085351 B (JAMES H. MARTIN) 04.02.1991 defines an adjustable dose dispenser having a plurality of different sized metering chambers with a stem rotatable to select the chamber to be discharged was disclosed. This device requires a separate metering chamber for each volume to be dispensed. Device has recesses and they are of different sizes and provide metering chambers of a plurality of different sizes. In order to adjust the amount or volume dispensed upon each actuation of the nozzle and actuator, it is merely necessary to rotate the nozzle and actuator. Suitable indicia are provided on the cap and on a depending flange portion on the nozzle and actuator member to facilitate such selection.

Dispensers can be produced according to US 5183187 B (JAMES H. MARTIN ET.AL.) 02.02.1993 which employs an arrangement utilizing a piston to achieve dispensing of a predetermined quantity of fluid. It has a single chamber where the volume dispense from the chamber is adjustable. To achieve adjustment of the dosage, rotation of stem member causes the threads to move section up and down. Thus changing the size of chamber and, hence, the amount of liquid is dispensed.

EP 1413529 B (MARTIN, JAMES H.) 28.04.2004 discloses an improved adjustable metering valve for dispensing pressurized liquids. The upper portion of the valve has a plurality of notches around the circumference and printed near each notch is a marking indicative of the dosage of liquid to be dispensed upon the depression of the actuator with the nozzle received into the associated notch.

According to this invention plurality of different sized metering chambers system or single chamber with a piston or any kind of dose adjustable dispenser can be used.

Dispenser can be suitable for pump spray or pump spray for oral administration or aerosol spray for oral administration.

Another object is to embrace a pump spray composition of rasagiline. In accordance with an embodiment, the pump spray delivers the equivalent of about 0.25 mg of rasagiline per 21 mcL actuation.

In yet another embodiment, the pump spray delivers the equivalent of about 0.50 mg of rasagiline per 42 mcL actuation.

In yet another embodiment, the pump spray delivers the equivalent of about 0.75 mg of rasagiline per 63 mcL actuation.

In yet another embodiment, the pump spray delivers the equivalent of about 1 mg of rasagiline per 84 mcL actuation.

Another object is to embrace an aerosol spray composition of rasagiline. In accordance with an embodiment, the aerosol spray delivers the equivalent of about 0.25 mg of rasagiline per 42 mcL actuation.

In yet another embodiment, the aerosol spray delivers the equivalent of about 0.50 mg of rasagiline per 84 mcL actuation.

In yet another embodiment, the aerosol spray delivers the equivalent of about 0.75 mg of rasagiline per 126 mcL actuation.

In yet another embodiment, the aerosol spray delivers the equivalent of about 1 mg of rasagiline per 168 mcL actuation.

Liquid composition containing a spray dispenser is typically administered to oral mucosal surface or added to other suitable liquids for oral ingestion such as, but not limited to, water, fruit juices, vegetable juices, soft drinks etc.

In a specific embodiment it is administered sublingually, supralingually or buccally.

Drug delivery through the oral mucosa avoids drug degradation in acidic medium of stomach and it is provided that patient compliance and convenience are enhanced.

The term "spray" as used herein encompasses a spray, a liquid, droplets, a mist, a fluid flow or any other liquid form for oral mucosal administration.

The term of "pump spray" covers a device suitable for dispensing a spray or mist such as a spray container, an atomizer and the like. Pump spray includes mechanically and/or air activated sprays.

The term of "rasagiline" covers pharmaceutically acceptable bases, salts, isomers, racemates, racemic mixtures, individual diasteriomers, enantiomers and the like.

In another aspect of this invention, rasagiline or pharmaceutically acceptable salt thereof can be solved through using of caprylocaproyl macrogol-8 glyceride, glyceryl mono- & dicaprate, polyoxyethylene sorbitan fatty acid esters, 2-(2-ethoxyethoxy)ethanol, polyoxyethylene castor oil derivatives, polyethylene glycol, propylene glycol,isopropyl alcohol, ethanol, methanol, n-butanol, acetone, ethyl methyl ketone, methyl isobutyl ketone, water,ethyl acetate, n-propyl acetate, n-butyl acetate, t-butyl acetate,acetonitrile, propionitrile or mixtures thereof and the like.

This invention provides a solution of rasagiline or pharmaceutically acceptable salt thereof in water or aqueous organic solvent or organic solvent to be used in a spray dispenser for oral administration. Rasagiline or pharmaceutically acceptable salt thereof can be dissolved or dispersed at a suitable temperature which does not affect its stability. The solution can optionally be filtered by passing through paper, glass fibre, or other membrane material such them.

In formulations of this invention, surfactant, solubilizer, micelle-creating agent, emulsifying agent, viscosity modifying agent, mixtures thereof and the like can be utilized.

Formulations of this invention may also contain buffering agents, sweetening, taste masking, antimicrobial or flavouring agents.

Buffering agent is but not limited to citric acid, sodium citrate, sodium phosphate, potassium citrate, mixtures thereof and the like.

Various flavors or flavouring agents may be used to mask the bitterness of the active ingredient. Flavors, are but not limited to bubble gum, cherry, fruit, honey, lemon, liquorice, mint, orange, peppermint, spearmint, wintergreen, mixtures thereof and the like.

Sweeteners, are but not limited to, corn syrup, dextrose, invert sugar, fructose, saccharin, aspartame, acesulfame-K, Stevia rebaudiana, sucralose, sorbitol, mannitol, zylitol, mixtures thereof and the like.

Preservatives are, but not limited to, sodium benzoate, benzoic acid, ethylenediaminetetraacetic acid, sorbic acid, benzethonium chloride, benzalkonium chloride, bronopol, butyl paraben, methyl paraben, ethylparaben, propyl paraben, thiomerosol, sodium propionate, chlorhexidine, chlorobutanol, chlorocresol, cresol, imidurea, phenol, phenylmercuric salts, potassium sorbate, propylene glycol, mixtures thereof and the like.

Ethanol and/or propylene glycol, benzyl alcohol, the parabens such as butylparaben-methylparaben, glycerol, propylene glycol, chlorobutanol, phenol, phenoxyethanol, and phenylethyl alcohol may also be used in formulations.

Viscosity modifying agents are but not limited to, polyvinyl alcohol, acacia, alginic acid, bentonite, carbomer, carboxymethylcellulose, carrageenan, cellulose, ceratonia, cetyl alcohol, chitosan, colloidal silicon dioxide, ethylcellulose, gelatin, guar gum, hydroxyethyl cellulose, hydroxyethylmethyl cellulose, hydroxypropyl cellulose, hypromellose, magnesium aluminum silicate, maltitol, maltodextrin, medium-chain triglycerides, methylcellulose, polydextrose, polyethylene oxide, povidone, propylene glycol alginate, sodium alginate, stearyl alcohol, sucrose, tragacanth, trehalose, wax, xanthan gum, mixtures thereof and the like.

Surfactants are, but not limited to, functionalized siloxanes, fluorinated and perfluorinated products such as perfluorinated alcohols, and polyoxyalkylenes such as the ethylene oxide and/or propylene oxide adducts of alkylphenols, the ethylene oxide and/or propylene oxide adducts of long chain alcohols or fatty acids, mixed ethylene oxide/propylene oxide block copolymers, sorbitan fatty acid esters, ethoxylated sorbitan fatty acid esters, polyethoxylated sorbitan fatty acid esters, ethoxylated alcohols fatty amine oxides, and glycerol esters, sorbitan monooleate, sorbitan sequioleate, sorbitan trioleate, polyoxyethylene sorbitan monooleate, sodium isostearyl-2-lactate,diblock and triblock copolymers based on polyester derivatives of fatty acids and poly[ethylene oxide], diblock and triblock copolymers based on polyisobutylene succinic anhydride and poly[ethylene oxide], reaction products of ethylene oxide and propylene oxide with ethylenediamine, alkyl polyoxyethylene sulfates, cholic acid and other bile acids (e.g., cholic acid, deoxycholic acid, glycocholic acid, taurocholic acid, glycodeoxycholic acid) and salts thereof (e.g., sodium deoxycholate, etc.), dioctyl sodium sulfosuccinate, glyceryl esters, phosphatide acid and their salts, potassium laurate, sodium alginate, sodium carboxymethylcellulose, sodium dodecylsulfate, and sodium lauryl sulphate, mixtures thereof, and the like.

Yet another object of the present invention is to provide an aerosol spray, which contains rasagiline, capable to emit a predetermined quantity wherein it has plurality of predetermined quantity of portions so as to adjust doses through plurality of spray settings. Aerosol spray package having more than one adjuster allows user to change the setting so as to produce different doses. Patients are able to select desired doses pursuant to dose titration.

Aerosol spray includes propellant-driven sprays.

Another embodiment of this invention covers pump spray formulation. Formulation for per pump spray comprises rasagiline or pharmaceutically acceptable salt thereof is from 1 % to 50 %, viscosity agent is from 1,5 % to 20 %, sweetener is from 1 % to 20 %, flavouring agent is from 2 % to 10 %, buffering agent(s) is from 0.05 % to 5 % and solvent is up to 90 % by weight of formulation.

Another embodiment of this invention is aerosol spray formulation of rasagiline or pharmaceutically acceptable salt thereof for oral administration.

Sprayed rasagiline or pharmaceutically acceptable salt thereof from canister may be completely dissolved or dispersed, namely aerosol spray formulation may be based on solution or suspension.

In accordance with this invention aerosol compositions contain a propellant and rasagiline and preferably excipient or mixtures of excipients for administration to mucosal surface or other suitable liquids for oral ingestion such as, but not limited to, water, fruit juices, vegetable juices, soft drinks etc.

The spray compositions of the invention are administered from a sealed and pressurized container.

Suitable propellants are, but not limited to, hydrocarbons (butane, propane, etc.), chlorofluorocarbons (CFC-II, CFC-12, etc.), hydrofluorocarbons (HFA-134a, HFA-227ea, etc.), ethers (dimethylether, diethylether, etc.), perfluorocarbons, mixtures thereof and the like.

Another embodiment of this invention covers aerosol spray formulation. Formulation for per canister comprises rasagiline or pharmaceutically acceptable salt thereof is from 0.1 % to 20 %, viscosity agent is from 0,5 % to 20 %, sweetener is from 0.5 % to 20 %, flavouring agent is from 0.1 % to 10%, buffering agent(s) is from 0.01 % to 2 % and solvent is up to 70 %, propellant is up to 95 % by weight of formulation.

### Example 1: Pump Spray Formulation

As an example of pump spray formulation and manufacturing method thereof of this invention, but not limited to, is given as following.

Unit formula of Example 1, ingredients per dispenser, is listed in Table 1.

**Table 1**

| Ingredient | Function | Quantity/Dispenser | % (w/v) |
|---|---|---|---|
| Rasagiline Mesylate | Active Substance | 46.8 mg | 1.872 |
| Glycerine | Sweetener / Viscosity Increasing Agent | 50.0 mg | 2.000 |
| Sucralose | Sweetener | 185.0 mg | 7.400 |
| Peppermint Oil | Flavouring Agent | 70.0 mg | 2.800 |
| Water | Solven/Vehicle | q.s. 2.5 mL | q.s. 100 % |

Manufacturing method of Example 1 is as following;

A) Rasagiline Mesylate (46.8 g), Sucralose (185 g) and Peppermint Oil (70 g) are added to water, dissolved and mixed for 15 minutes to assure complete dissolution.

B) Glycerine (50 g) is added to solution prepared in Step A, and mixed for 15 minutes.

C) Water is added to solution prepared in Step B to complete the volume to 2.5 L and mixed for 30 minutes.

D) Solution prepared in Step C is filled into PE or Type III glass bottles as 2.75 mL±5% per bottle.

### Example 2: Aerosol Spray Formulation

As an example of aerosol formulation and manufacturing method thereof of this invention, but not limited to, is given as following.

Unit formula of Example 2, ingredients per dispenser, is listed in Table 2. **Table 2**

| Ingredient | Function | Quantity/Dispenser | % (w/v) |
|---|---|---|---|
| Rasagiline Mesylate | Active Substance | 46.8 mg | 0.936 |
| Propylene Glycol | Antimicrobial preservative/ Vehicle for Flavour | 615 mg | 12.300 |
| Acesulfame K | Sweetener | 55 mg | 1.100 |
| Watermelon Flavour | Flavouring Agent | 75 mg | 1.500 |
| Water | Solven/Vehicle | 1500 mg | 30.00 |
| HFA-134a | Propellant | q.s. 5 mL | q.s. 100 % |

Manufacturing method of Example 2 is as following;

A) Rasagiline Mesylate (93.6 g), Acesulfame K (110 g), Watermelon Flavour, propylene glycol (1380 g) are added to water, dissolved and mixed for 25 minutes to assure complete dissolution.

B) Remaining water is added to solution prepared in Step B and mixed for 30 minutes.

C) Solution prepared in Step B is mixed with propellant, HFA-134a, and filled into canisters as 7.5 mL±5% per canister.

## Claims

1. Aerosol spray or pump spray of rasagiline or pharmaceutically acceptable salt thereof for oral administration.

2. Aerosol spray or pump spray, as claimed in claim 1, contains rasagiline or pharmaceutically acceptable salt thereof which is dispersed or dissolved therein.

3. Dispersion or solution, as claimed in claim 2, is prepared through using of solvents selected from the group consisting of caprylocaproyl macrogol-8 glyceride, glyceryl mono- & dicaprate, polyoxyethylene sorbitan fatty acid esters, 2-(2-ethoxyethoxy)ethanol, polyoxyethylene castor oil derivatives, polyethylene glycol, propylene glycol,isopropyl alcohol, ethanol, methanol, n-butanol, acetone, ethyl methyl ketone, methyl isobutyl ketone, water,ethyl acetate, n-propyl acetate, n-butyl acetate, t-butyl acetate,acetonitrile, propionitrile or mixtures thereof and the like.

4. Per pump spray formulation, as claimed in claim 1, comprising rasagiline or pharmaceutically acceptable salt thereof is from 1 % to 50 %, viscosity agent is from 1,5 % to 20 %, sweetener is from 1 % to 20 %, flavouring agent is from 2% to 10%, buffering agent(s) is from 0.05 % to 5 % and solvent is up to 90 % by weight of formulation.

5. Per aerosol spray formulation, as claimed in claim 1, comprising rasagiline or pharmaceutically acceptable salt thereof is from 0.1 % to 20 %, viscosity agent is from 0,5 % to 20 %, sweetener is from 0.5 % to 20 %, flavouring agent is from 0.1 % to 10 %, buffering agent(s) is from 0.01 % to 2 % and solvent is up to 70 %, propellant is up to 95 % by weight of formulation.

6. Propellant, as claimed in claim 5, is selected from group consisting of hydrocarbons or chlorofluorocarbons or hydrofluorocarbons or ethers or perfluorocarbons or mixtures thereof.

7. Pump or aerosol spray formulation, as claimed in preceding claims, is contained in an aerosol spray canister or pump spray dispenser **characterized by** that said canister or dispenser has plurality of spray settings for different doses.

8. Aerosol spray or pump spray, as claimed in claim 7, has more than one dose indicator as 0.25 mg, 0.5 mg, 0.75 mg and 1 mg pursuant to dose titration procedure.

9. Each of dose indicators, as claimed in claim 7 and claim 8, is coloured differently from each other.

10. According to preceding claims: pump spray delivers the equivalent of 0.25 mg of rasagiline per 21 mcL actuation or the pump spray delivers the equivalent of 0.50 mg of rasagiline per 42 mcL actuation or the pump spray delivers the equivalent of 0.75 mg of rasagiline per 63 mcL actuation or the pump spray delivers the equivalent of 1 mg of rasagiline per 84 mcL actuation.

11. According to preceding claims: aerosol spray delivers the equivalent of 0.25 mg of rasagiline per 42 mcL actuation or aerosol spray delivers the equivalent of 0.50 mg of rasagiline per 84 mcL actuation or aerosol spray delivers the equivalent of 0.75 mg of rasagiline per 126 mcL actuation or aerosol spray delivers the equivalent of 1 mg of rasagiline per 168 mcL actuation.
